# EUROPÄISCHE PATENTSCHRIFT

(11) **EP 1 592 694 B1**
(45) Veröffentlichungstag und Bekanntmachung des Hinweises auf die Patenterteilung: **02.05.2007**
(21) Anmeldenummer: 04710847.7
(22) Anmeldetag: 13.02.2004
(51) Int. Cl.: C07D 495/04, A61K 31/435

(54) **WIRKSTOFFPARTIKEL MIT CLOPIDOGREL ODER EINEM PHARMAZEUTISCH VERTRÄGLICHEN SALZ DAVON**
ACTIVE INGREDIENT PARTICLES WITH CLOPIDOGREL OR A PHARMACEUTICALLY ACCEPTABLE SALT THEREOF
PARTICULES DE SUBSTANCE ACTIVE AVEC CLOPIDOGREL OU UN SEL DE CELUI-CI PHARMACEUTIQUEMENT ACCEPTABLE

(30) Priorität: 13.02.2003 DE 10305984
(43) Veröffentlichungstag der Anmeldung: 09.11.2005
(60) Teilanmeldung: 05009789.8
(73) Patentinhaber: Helm AG, 20097 Hamburg (DE)
(72) Erfinder: DOSER, Karlheinz, 21244 Buchholz i.d.Nordheide (DE); GLÄNZER, Klaus, 22337 Hamburg (DE)
(74) Vertreter: Teipel, Stephan
(86) Internationale Anmeldenummer: PCT/EP2004/001370
(87) Internationale Veröffentlichungsnummer: WO 2004/072085

(56) Entgegenhaltungen:
- EP-A- 1 161 956
- WO-A-20/04013147
- US-A- 4 847 265
- US-B1- 6 284 277
- US-B1- 6 509 348

## Beschreibung

Die vorliegende Erfindung betrifft Wirkstoffpartikel mit Clopidogrel oder einem pharmazeutisch verträglichen Salz davon.

Clopidogrel (5-Methyl-α-(4,5,6,7-tetrahydro[2,3-c]thienopyridyl)(2-chlorphenyl)acetat) ist als Wirkstoff aus EP-A-0 099 802 bekannt. Clopidogrel wirkt als Plättchenaggregationshemmer und kann daher beispielsweise zur Prävention thromboembolischer Ereignisse, wie z.B. Schlaganfall oder Myokardinfarkt, eingesetzt werden.

Die EP-A-0 281 459 schlägt vor, in pharmazeutischen Formulierungen anorganische Salze des (S)-(+)-Clopidogrels einzusetzen, insbesondere (S)-(+)-Clopidogrel-Hydrogensulfat. Dieses Dokument offenbart auch organische Salze des Clopidogrels, diese werden jedoch als amorph und/oder hygroskopisch sowie schwer zu reinigen beschrieben.

Das in pharmazeutischen Formulierungen eingesetzte (S)-(+)-Clopidogrel-Hydrogensulfat weist den Nachteil auf, dass zu seiner Herstellung konzentrierte Schwefelsäure benötigt wird und dass entsprechende Produkte wegen des aziden Protons stark sauer reagieren. Diese sauren Eigenschaften beeinflussen die Kompatibilität mit vielen pharmazeutischen Hilfsstoffen und damit die Stabilität entsprechender Arzneiformen negativ. Es besteht daher ein Bedürfnis nach stabilen, leicht zu reinigenden und leicht mit verschiedenen pharmazeutischen Hilfsstoffen, wie Arzneimittelträgern und -zusatzstoffen, verarbeitbaren Formen von Clopidogrel.

Eine Aufgabe der vorliegenden Erfindung besteht darin, Clopidogrel oder ein pharmazeutisch verträgliches Salz davon in einer einfach weiterverarbeitbaren Form zur Verfügung zu stellen. Dies wird erfindungsgemäß dadurch erreicht, dass das Salz auf ein festes Adsorbtionsmittel aufgebracht wird. Hierdurch werden Wirkstoffpartikel erhalten, die sich leicht schütten und dosieren lassen.

Als Adsorbtionsmittel eignet sich jeder physiologisch und pharmazeutisch akzeptable, vorzugsweise partikuläre Feststoff, der in der Lage ist, Clopidogrel oder ein Salz davon zu adsorbieren. Vorzugsweise ist der Feststoff ein rieselfähiges Pulver, das leicht zu oralen pharmazeutischen Formulierungen weiterverarbeitet werden kann.

Physiologisch und pharmazeutisch akzeptable Adsortionsmittel sind beispielsweise:
1. Natürliche oder aufbereitete Adsorbtionsmittel aus der Gruppe der Tonerden (Tonmaterialien) und sonstiger Erden und Mineralien, z.B. Attapulgite, Aluminium-Magnesium-Silikate (Carrisorb^{®}, Gelsorb^{®}), Magnesium-Aluminium-Silikate (Pharmasorb^{®}, Veegum^{®}), Magnesiumsilikate (Talkum), Calciumsilikate, Bentonite, Kaolin, Magnesium-Trisilikate, Montmorillonite, Porzellanerden (Bolus), Sepiolite (Meerschaum)
2. Kieselgele, Kieselgur, Kieselsäuren
3. Kolloidale (hochdisperse) Kieselsäuren (hydrophobe oder hydrophile Aerosile^{®}, Cabo-sile^{®})
4. Cellulosen, modifizierte Cellulosen, fein- und mikrokristalline Cellulosen sowie Cellulosederivate, Celluloseacetat, Cellulosefettsäureester, Cellulosenitrate, Celluloseether (Carboxymethylcellulosen, Ethylcellulosen, Hydroxyethylcellulosen, Hydroxypropylcellulosen, Methylcellulosen, Methylethylcellulosen, Methylhydroxypropylcellulosen)
5. Zucker und Zuckerderivate (Mono- und Polysaccharide), Lactosen, Dextrane, Dextrose, Cyclodextrine
6. Native Mais-, Reis-, Tapioka-, Weizen-, Kartoffelstärken und deren Derivate, Dextrine, prägelatinisierte, ganz oder teilweise hydrolysierte Stärken
7. Feste Polyole, insbesondere Mannit oder Sorbit
8. Polyacrylate, Acrylsäurepolymerisate bzw. Copolymerisate
9. Phosphate, Sulfate, Carbonate, Gluconate, Oxide von Alkali- und Erdalkalimetallen sowie physiologisch akzeptablen Schwer- und Übergangsmetallen
10. Guar-Mehl, Guar-Gummi
11. Johannisbrotmehl (Carob-Mehl, Carob-Gummi)
12. Alginsäure, Alginate und Algenmehl
13. Tragant
14. Carbo vegetabilis (Kohle)
15. Pektine und Amylopektine
16. N-Vinylpyrrolidon-Polymere, wie z.B. Povidon oder Crospovidon.

Die Adsorbtionsmittel können einzeln oder in Mischung aus zwei oder mehr Adsorbtionsmitteln eingesetzt werden. Außerdem können die erfindungsgemäßen Wirkstoffpartikel neben dem Adsorbtionsmittel übliche pharmazeutische Hilfsstoffe beispielsweise zur Herstellung von Direkttablettiermischungen bzw. zur Herstellung von Granulaten zur Weiterverarbeitung zu Arzneimitteln umfassen. Alternativ können die erfindungsgemäßen Wirkstoffpartikel nach ihrer Herstellung mit entsprechenden Hilfsstoffen vermischt und dann zu pharmazeutischen Formulierungen weiterverarbeitet werden.

Besonders bevorzugte Adsorbtionsmittel sind bestimmte Lactosen (z. B. Lactopress^{®}), bestimmte Mannite (z. B. Mannogem^{®}) und bestimmte Cellulosen (z. B. Celphere^{®}), insbesondere Lactopress^{®}. Ein Granulat auf Basis von pyrogen hergestelltem Siliciumdioxid wird, obwohl möglich, vorzugsweise nicht als Trägermedium eingesetzt.

Zur Desorptionssteuerung können geeignete Netzmittel eingesetzt werden. Zur Stabilitätsverbesserung können beispielsweise Antioxidantien, wie z.B. Ascorbinsäure und deren Salze, zugefügt werden. Weitere geeignete Hilfsmittel sind Emulgatoren, Lösungsmittel und Lösungsvermittler.

Die erfindungsgemäßen Wirkstoffpartikel können beispielsweise aus einem Lösungsmittel gewonnen werden, in dem das Adsorbtionsmittel unlöslich oder schwer löslich und das Clopidogrel oder das Salz davon löslich ist. Hierzu kann das Adsorbtionsmittel in dem Lösungsmittel suspendiert werden. Vor oder nach dem Suspendierungsschritt kann das Clopidogrel oder das Salz davon in dem Lösungsmittel gelöst werden. Der Wirkstoff kann dabei entweder direkt oder als Lösung in demselben oder einem anderen Lösungsmittel zugegeben werden. Anschließend werden die Wirkstoffpartikel, die das Clopidogrel oder das Salz davon auf dem Adsorbtionsmittel aufgebracht umfassen, aus dem Lösungsmittel beispielsweise durch Verdampfen des Lösungsmittels gewonnen.

Als Lösungsmittel eignen sich alle üblichen Lösungsmittel, in denen das gewählte Adsorbtionsmittel nicht löslich oder schwer löslich und das Clopidogrel oder das Salz davon löslich ist. Beispielsweise können die vorstehend zur Herstellung des Salzes beschriebenen Lösungsmittel verwendet werden.

In einer alternativen Ausführungsform des erfindungsgemäßen Verfahrens zur Herstellung von Wirkstoffpartikeln führt man die letzte Stufe der Synthese von Clopidogrel in Gegenwart des Adsorbtionsmittels durch. Dadurch können die gewünschten Wirkstoffpartikel ohne einen isolierenden Zwischenschritt hergestellt werden. Ferner können beispielsweise Clopidogrel und eine Säure mit der Suspension des Adsorbtionsmittels vermischt werden. Dabei können das Clopidogrel und die Säure jeweils getrennt in einem Lösungsmittel aufgelöst und gleichzeitig oder nacheinander zu der Suspension zugegeben werden. Alternativ können das Clopidogrel und die Säure in reiner Form zu der Suspension zugegeben werden. Einzelne Bestandteile können auch separat vorgemischt und dann gemeinsam zu der Suspension zugegeben werden.

Das Gewichtsverhältnis von Adsorbtionsmittel zu darauf adsorbiertem Clopidogrel oder einem Salz davon ist für die vorliegende Erfindung nicht besonders wesentlich und kann vom Fachmann in Abhängigkeit von dem gewünschten Verwendungszweck frei gewählt werden. Bei einer Weiterverarbeitung zu oralen, pharmazeutischen Formulierungen sollte jedoch beachtet werden, dass genügend Clopidogrel auf dem Adsorbtionsmittel aufgebracht ist, damit die gewünschte Dosierung in der Einheitsdosisform erreicht wird. Beispielsweise kann das Gewichtsverhältnis von Clopidogrel oder dem Salz des Clopidogrels, bezogen auf die freie Clopidogrel-Base, zu Adsorbtionsmittel im Bereich von 2:1 bis 1:6 liegen (d.h. z.B. 1 Gewichtsteil Clopidogrel-Base pro 6 Gewichtsteile Adsorbtionsmittel), vorzugsweise im Bereich von 1:1 bis 1:3.

Bevorzugte Salze des Clopidogrels sind Hydrogensulfat, Hydrochlorid, Mesylat, Besylat, Tosylat und Napsylat.

Die vorliegende Erfindung wird durch die nachfolgenden Beispiele näher erläutert, ohne sie auf diese einzuschränken.

### Beispiel 1

### Adsorbat von (S)-(+)-Clopidogrel-Besylat an Calciumgluconat als Trägermaterial

Zu einer Lösung von 19,7 g (61,4 mmol) (S)-(+)-Clopidogrel in 300 ml wasserfreiem Diethylether wird bei 3° C langsam (ca. 30 min.) unter starkem Rühren eine Lösung von 11 g (69,5 mmol) wasserfreier Benzolsulfonsäure in 100 ml kalten, wasserfreien Diethylether getropft. Anschließend wird eine vorbereitete Aufschlämmung von 28 g Calciumgluconat in kaltem, wasserfreien Diethylether langsam zugegeben. Der nach Beendigung der Zugabe entstandene Kristallbrei wird abgenutscht, mit eiskaltem, wasserfreien Diethylether gewaschen und anschließend getrocknet.

Man erhält ein weißes, rieselfähiges Pulver.

### Beispiel 2

### Adsorbat von (S)-(+)-Clopidogrel-Besylat an Kieselgel/Mannit als Trägermaterial

Bei einer Temperatur von 2° - 3° C werden 20 g (62,3 mmol) (S)-(+)-Clopidogrel und 11 g (69,5 mmol) wasserfreie Benzolsulfonsäure in 200 ml wasserfreiem Diethylether zur Reaktion gebracht. Anschließend wird eine Aufschlämmung von 2 g Kieselsäure und 20 g Mannit in 100 ml wasserfreiem Diethylether langsam zugegeben. Das entstandene Adsorbat wird in der Kälte abgenutscht, mit eiskaltem, wasserfreien Diethylether gewaschen und anschließend getrocknet.

Man erhält 39 g eines weißen, rieselfähigen Pulvers.

### Beispiel 3

### Adsorbat von (S)-(+)-Clopidogrel-Mesylat an Kieselgel/Mannit als Trägermaterial

Zu einer Lösung von 19,5 g (60,7 mmol) (S)-(+)-Clopidogrel in 300 ml wasserfreiem Diethylether wird bei 3°C langsam (ca. 30 min.) eine Lösung von 5,85 g (60,8 mmol) wasserfreier Methansulfonsäure in 100 ml kaltem, wasserfreiem Diethylether getropft. Anschließend wird eine vorbereitete Aufschlämmung von 1,95 g Kieselsäure und 19,5 g Mannit in kaltem, wasserfreiem Diethylether langsam zugegeben. Das nach Beendigung der Zugabe entstandene Adsorbat wird abgenutscht, mit eiskaltem, wasserfreien Diethylether gewaschen und anschließend getrocknet.

Man erhält 30 g eines rieselfähigen, weißen Pulvers.

### Beispiel 4

### Adsorbat von (S)-(+)-Clopidogrel-Mesylat an Mannit als Trägermaterial

19,5 g (60,7 mmol) (S)-(+)-Clopidogrel und 5,85 g (60,8 mmol) Methansulfonsäure werden analog zum Beispiel 3 zur Reaktion gebracht. Anschließend wird eine vorbereitete Aufschlämmung von 19,5 g Mannit in kaltem, wasserfreien Diethylether langsam zugegeben. Das nach Beendigung der Zugabe entstandene Adsorbat wird abgenutscht, mit eiskaltem, wasserfreien Diethylether gewaschen und anschließend getrocknet.

Man erhält 29,7 g eines rieselfähigen, weißen Pulvers.

### Beispiel 5

### Adsorbat von (S)-(+)-Clopidogrel an Kieselgel/Maisstärke als Trägermaterial

Zu einer Suspension von 2 g Aerosil 200 in CH₂Cl₂ wird eine Lösung von 5 g (15,6 mmol) (S)-(+)-Clopidogrel in wasserfreiem Dichlormethan langsam zugetropft. Nach 1 h wird eine Suspension von 4 g gelatinisierter Maisstärke in wasserfreiem Dichlormethan unter Rühren zugegeben. Nach Beendigung der Zugabe wird das Lösungsmittel abgezogen, wobei ein rein weißer Feststoff erhalten wird, der anschließend 12 h im Vakuum getrocknet wird.

Man erhält ein rein weißes, rieselfähiges Pulver mit einer Wirkstoffbeladung von 45,5%.

Die Wiederholung des Versuchs unter Verwendung von 8 g gelatinisierter Maisstärke ergibt ein rieselfähiges Pulver mit einer Wirkstoffbeladung von 33,3%.

### Beispiel 6

Es wurden zwei verschiedene Verfahren zur Herstellung von Adsorbaten der Clopidogrel-Salze angewandt. Im ersten Verfahren wird das Salz in einem geeigneten Lösungsmittel gelöst und in dieser Lösung wird das Adsorbtionsmittel suspendiert.

In einer zweiten Versuchsreihe wurde die Clopidogrel-Base in einem geeigneten Lösungsmittel gelöst, das Adsorbtionsmittel wurde zugegeben und das Salz auf das Trägermaterial ausgefällt.

In allen Versuchen wurden jeweils Lactose (Lactopress^{®}), Mannit (Mannogem^{®}) und Cellulose (Celphere^{®}) als Adsorbtionsmittel eingesetzt.

### Folgende Versuche wurden durchgeführt:

### Clopidogrel-Salz-Adsorbate mit Isolierung des Salzes

### a) Clopidogrel-Besylat-Adsorbate

Es werden 1,5 g (3,1 mmol) Clopidogrel-Besylat in 20 ml Aceton gelöst und 1,5 g Adsorbtionsmittel zugegeben. Das Lösungsmittel wird abgezogen, der Rückstand kurz mit MTB-Ether aufgeschlämmt und anschließend im Vakuum getrocknet.

### b) Clopidogrel-Hydrochlorid-Adsorbate

500 mg (1,4 mmol) Clopidogrel-Hydrochlorid werden in 10 ml Aceton gelöst. 500 mg Adsorbtionsmittel werden zugegeben und verrührt. Das Lösungsmittel wird abgezogen. Der Rückstand wird im Vakuum getrocknet.

### c) Clopidogrel-Hydrogensulfat-Adsorbate

500 mg (1,2 mmol) Clopidogrel-Hydrogensulfat werden in 10 ml Aceton gelöst. 500 mg Adsorbtionsmittel werden zugegeben und verrührt. Das Lösungsmittel wird abgezogen. Der Rückstand wird im Vakuum getrocknet.

### Clopidogrel-Salz-Adsorbate ohne vorherige Isolierung der Salze

### 1. Lösungsmittel Diethylether

### a) Clopidogrel-Besylat-Adsorbate

4,018 g (12,5 mmol) Clopidogrel-Base werden in 20 ml Diethylether gelöst. 6 g Adsorbtionsmittel und 1,977 g (12,5 mmol) Benzolsulfonsäure werden in 20 ml Ether zugegeben. Das Festprodukt wird abgesaugt, mit Ether gewaschen und im Vakuum getrocknet.

### b) Clopidogrel-Mesylat-Adsorbate

4,018 g (12,5 mmol) Clopidogrel-Base werden in 20 ml Diethylether gelöst. 6 g Adsorbtionsmittel und 1,2 g (12,5 mmol) Methansulfonsäure werden in 20 ml Ether zugegeben. Das Festprodukt wird abgesaugt, mit Ether gewaschen und im Vakuum getrocknet.

### c) Clopidogrel-Hydrochlorid-Adsorbate

3 g (9,3 mmol) Clopidogrel-Base werden in 31 ml Diethylether gelöst. 3 g Adsorbtionsmittel werden zugegeben und Chlorwasserstoffgas wird eingeleitet. Das Festprodukt wird abgesaugt, mit Ether gewaschen und im Vakuum getrocknet.

### 2. Lösungsmittel Methyl-tert-butylether (MTB-Ether)

### a) Clopidogrel-Besylat-Adsorbate

4,018 g (12,5 mmol) Clopidogrel-Base werden in 40 ml MTB-Ether gelöst. 6 g Adsorbtionsmittel und 1,977 g (12,5 mmol) Benzolsulfonsäure werden in 50 ml MTB-Ether zugegeben. Das Festprodukt wird abgesaugt, mit MTB-Ether gewaschen und im Vakuum getrocknet.

### b) Clopidogrel-Mesylat-Adsorbate

4,018 g (12,5 mmol) Clopidogrel-Base werden in 40 ml MTB-Ether gelöst. 6 g Adsorbtionsmittel und 1,2 g (12,5 mmol) Methansulfonsäure werden in 50 ml MTB-Ether zugegeben. Das Festprodukt wird abgesaugt, mit MTB-Ether gewaschen und im Vakuum getrocknet.

### Beispiel 7

Die Stabilität der gemäß Beispiel 6 erhaltenen Adsorbate wurde untersucht. Die Adsorbate bleiben bei Raumtemperatur pulverig und verändern ihre Farbe über mehr als zwei Monate nicht.

Die Abnahme des Wirkstoffgehalts bei einer Lagerung über 15 Tage bei 40° bzw. 60° C und 75% relativer Luftfeuchtigkeit wurde gemessen. Die Ergebnisse sind in der nachfolgenden Tabelle 1 [Gehalt nach 15 Tagen (Anfangswert normiert auf 100%)] zusammengefasst.

**Tabelle 1**

| **Clopidogrel-Hydrochlorid** | **40°C** | **60°C** |
|---|---|---|
| Reines Salz | 93,66 | 42,54 |
| Lactopress | 99,78 | 54,89 |
| Celpher | 92,81 | 43,74 |
| | | |

| **Clopidogrel-Mesylat** | **40° C** | **60°C** |
|---|---|---|
| Reines Salz | 97,56 | 17,11 |
| Lactopress | 83,33 | 59,39 |
| Mannogem | 105,51 | 21,76 |
| | | |

| **Clopidogrel-Besylat** | **40°C** | **60°C** |
|---|---|---|
| Reines Salz | 103,32 | 66,48 |
| Lactopress/Diethylether | 106,91 | 94,47 |
| Lactopress/MTB-Ether | 94,74 | 92,58 |

Man erkennt, dass die Adsorbate gegenüber den freien Salzen insbesondere bei erhöhter Temperatur eine größere Stabilität aufweisen.

### Beispiel 8

Gemäß Beispiel 6 hergestellte Adsorbate können direkt zu Tabletten verpresst werden. Dies wird durch die nachfolgenden Beispielformulierungen verdeutlicht. Die verwendeten Mengen der in den nachfolgenden Beispielen angegebenen weiteren Hilfsstoffe sind dem Fachmann aufgrund seines Basiswissens bekannt und können Standardwerken zur Formulierung von Tabletten, wie z.B. Ritschel et al., "Die Tablette", Editio Cantor - Aulendorf, 2. Aufl., 2002, entnommen werden.

### a) Clopidogrel-Besylat-Mikrokristalline Cellulose-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Besylat -Mikrokristalline Cellulose-Adsorbat 219,54 mg (entspricht 75 mg Clopidogrel Base)
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 101 N
■ Abrieb: 0,11%
■ Zerfallzeit: 65 Sek.
■ Freisetzung: 100% nach 30 Min.

Die so erhaltenen Tabletten können auch mit einem Überzug wie z.B. einem enterischen Überzug oder einem Überzug zur Geschmacksmaskierung versehen werden.

### b) Clopidogrel-Besylat-Mannit Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Besylat-Mannit-Adsorbat (entspricht 75 mg Clopidogrel Base) 219,54 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 106 N
■ Abrieb: 0,15%
■ Zerfallzeit: 62 Sek.
■ Freisetzung: 100% nach 30 Min.

Die so erhaltenen Tabletten können mit einem Überzug wie z.B. einem enterischen Überzug oder einem Überzug zur Geschmacksmaskierung versehen werden.

### c) Clopidogrel-Besylat-Lactose-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Besylat-Lactose-Adsorbat (entspricht 75 mg Clopidogrel Base) 219,54 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 96 N
■ Abrieb: 0,21%
■ Zerfallzeit: 76 Sek.
■ Freisetzung: 100% nach 30 Min.

Die so erhaltenen Tabletten können mit einem Überzug wie z.B. einem enterischen Überzug oder einem Überzug zur Geschmacksmaskierung versehen werden.

### d) Clopidogrel-Mesylat-Mannitol-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Mesylat-Mannitol-Adsorbat (entspricht 75 mg Clopidogrel Base) 194,79 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 98 N
■ Abrieb: 0,21%
■ Zerfallzeit: 55 Sek.
■ Freisetzung: 100% nach 30 Min.

Die so erhaltenen Tabletten können mit einem Überzug wie z.B. einem enterischen Überzug oder einem Überzug zur Geschmacksmaskierung versehen werden.

### e) Clopidogrel-Mesylat-Lactose-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Mesylat-Lactose-Adsorbat 194,79 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 88 N
■ Abrieb: 0,22%
■ Zerfallzeit: 72 Sek.
■ Freisetzung: 100% nach 30 Min.

Die so erhaltenen Tabletten können mit einem Überzug wie z.B. einem enterischen Überzug oder einem Überzug zur Geschmacksmaskierung versehen werden.

### f) Clopidogrel-HCl-Lactose-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-HCI-Lactose-Adsorbat 167,0 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 95 N
■ Abrieb: 0,20%
■ Zerfallzeit: 75 Sek.
■ Freisetzung: 100% nach 30 Min.

Die so erhaltenen Tabletten können mit einem Überzug wie z.B. einem enterischen Überzug oder einem Überzug zur Geschmacksmaskierung versehen werden.

### g) Clopidogrel-HCl-Mikrokristalline Cellulose-Adsorbat

Aus dem Adsorbat wurden Clopidgrel-Tabletten mit einer Gesamtmasse von 275 mg mittels Direktverpressung nach folgender Zusammensetzung hergestellt:
■ Clopidogrel-HCl -Mikrokristalline Cellulose-Adsorbat (entspricht 75 mg Clopidogrel Base) 167,0 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 100 N
■ Abrieb: 0,13%
■ Zerfallzeit: 65 Sek.
■ Freisetzung: 100% nach 30 Min.

Die so erhaltenen Tabletten können auch mit einem Überzug wie z.B. einem enterischen Überzug oder einem Überzug zur Geschmacksmaskierung versehen werden.

### h) Clopidogrel-Hydrogensulfat-Mikrokristalline Cellulose-Adsorbat

Aus dem Adsorbat wurden Clopidgrel-Tabletten mit einer Gesamtmasse von 275 mg mittels Direktverpressung nach folgender Zusammensetzung hergestellt:
■ Clopidogrel-Hydrogensulfat -Mikrokristalline Cellulose-Adsorbat (entspricht 75 mg Clopidogrel Base) 195,75 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 108 N
■ Abrieb: 0,12%
■ Zerfallzeit: 78 Sek.
■ Freisetzung: 98% nach 30 Min.

Die so erhaltenen Tabletten können auch mit einem Überzug wie z.B. einem enterischen Überzug oder einem Überzug zur Geschmacksmaskierung versehen werden.

### i) Clopidogrel-Hydrogrensulfat-Mannitol-Adsorbat

Aus dem Adsorbat wurden Clopidgrel-Tabletten mit einer Gesamtmasse von 275 mg mittels Direktverpressung nach folgender Zusammensetzung hergestellt:
■ Clopidogrel-Hydrogensulfat -Mannitol-Adsorbat (entspricht 75 mg Clopidogrel Base) 195,75 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 110 N
■ Abrieb: 0,13%
■ Zerfallzeit: 80 Sek.
■ Freisetzung: 98% nach 30 Min.

Die so erhaltenen Tabletten können auch mit einem Überzug wie z.B. einem enterischen Überzug oder einem Überzug zur Geschmacksmaskierung versehen werden.

### j) Clopidogrel-Hydrogensulfat-Lactose-Adsorbat

Aus dem Adsorbat wurden Clopidgrel-Tabletten mit einer Gesamtmasse von 275 mg mittels Direktverpressung nach folgender Zusammensetzung hergestellt:
■ Clopidogrel-Hydrogensulfat -Lactose-Adsorbat (entspricht 75 mg Clopidogrel Base) 195,75 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 109 N
■ Abrieb: 0,13%
■ Zerfallzeit: 80 Sek.
■ Freisetzung: 98% nach 30 Min.

Die so erhaltenen Tabletten können auch mit einem Überzug wie z.B. einem enterischen Überzug oder einem Überzug zur Geschmacksmaskierung versehen werden.

## Patentansprüche

1. Wirkstoffpartikel, umfassend ein-festes-Adsorbtionsmittel und darauf adsorbiertes Clopidogrel oder ein pharmazeutisch verträgliches Salz davon.

2. Wirkstoffpartikel nach Anspruch 1, wobei das Salz ausgewählt ist aus der Gruppe bestehend aus Hydrogensulfat, Hydrochlorid, Mesylat, Besylat, Tosylat und Napsylat.

3. Wirkstoffpartikel nach Anspruch 1 oder 2, wobei das Adsorbtionsmittel Lactopress ist.

4. Verwendung von Wirkstoffpartikeln nach Anspruch 1, 2 oder 3 zur Herstellung einer pharmazeutischen Formulierung.

5. Pharmazeutische Formulierung, umfassend Wirkstoffpartikel nach Anspruch 1, 2 oder 3.

6. Verfahren zur Herstellung von Wirkstoffpartikeln wie in Anspruch 1, 2 oder 3 definiert, umfassend das Gewinnen der Wirkstoffpartikel aus einem Lösungsmittel, in dem das Adsorbtionsmittel unlöslich oder schwer löslich und das Clopidogrel oder das Salz davon löslich ist.

7. Verfahren nach Anspruch 6, umfassend das Suspendieren des Adsobtionsmittels in dem Lösungsmittel, das Lösen des Clopidogrels oder des Salzes davon in dem Lösungsmittel und das Gewinnen der Wirkstoffpartikel.

8. Verfahren nach Anspruch 6 oder 7, wobei die Wirkstoffpartikel durch Verdampfen des Lösungsmittels gewonnen werden.

9. Verfahren nach einem der Ansprüche 6 - 8, wobei Clopidogrel und eine Säure mit der Suspension des Adsorbtionsmittels vermischt werden.

10. Verfahren nach Anspruch 6, wobei die letzte Stufe der Synthese von Clopidogrel oder einem pharmazeutisch verträglichen Salz davon in Gegenwart des Adsorbtionsmittels durchgeführt wird.

## Claims

1. Active ingredient particles comprising a solid adsorbent and clopidogrel or a pharmaceutically acceptable salt thereof adsorbed thereon.

2. Active ingredient particles according to claim 1 wherein the salt is selected from the group consisting of hydrogen sulfate, hydrochloride, mesylate, besylate, tosylate and napsylate.

3. Active ingredient particles according to claim 1 or 2 wherein the adsorbent is Lactopress.

4. The use of active ingredient particles according to claim 1, 2 or 3 for preparing a pharmaceutical formulation.

5. A pharmaceutical formulation comprising active ingredient particles according to claim 1, 2 or 3.

6. A method for preparing active ingredient particles as defined in claim 1, 2 or 3, comprising the recovery of the active ingredient particles from a solvent in which the adsorbent is insoluble or poorly soluble and the clopidogrel or the salt thereof is soluble.

7. A method according to claim 6 comprising suspending the adsorbent in the solvent, dissolving the clopidogrel or the salt thereof in the solvent and recovering the active ingredient particles.

8. A method according to claim 6 or 7 wherein the active ingredient particles are recovered by evaporation of the solvent.

9. A method according to any of the claims 6 to 8 wherein the clopidogrel and an acid are mixed with the suspension of the adsorbent.

10. A method according to claim 6 wherein the last stage of the synthesis of clopidogrel or a pharmaceutically acceptable salt thereof is carried out in the presence of the adsorbent.

## Revendications

1. Particules de principe actif comprenant un agent d'adsorption solide et du clopidogrel adsorbé sur celui-ci ou un sel pharmaceutique toléré de celui-ci.

2. Particules de principe actif selon la revendication 1, le sel étant sélectionné dans le groupe composé d'hydrogénosulfate, d'hydrochlorure, de mésylate, de bésylate, de tosylate et de napsylate.

3. Particules de principe actif selon l'une des revendications 1 ou 2, l'agent d'adsorption étant le lactopress.

4. Utilisation de particules de principe actif selon l'une des revendications 1, 2 ou 3 pour la préparation d'une formulation pharmaceutique.

5. Formulation pharmaceutique comprenant des particules de principe actif selon l'une des revendications 1, 2 ou 3.

6. Procédé de préparation de particules de principe actif selon la définition des revendications 1, 2 ou 3, comprenant l'extraction de particules de principe actif d'un solvant dans lequel l'agent d'adsorption est insoluble ou difficilement soluble et le clopidogrel ou le sel de celui-ci sont solubles.

7. Procédé selon la revendication 6 comprenant la mise en suspension de l'agent d'adsorption dans le solvant, la dissolution du clopidogrel ou du sel de celui-ci dans le solvant et l'extraction des particules de principe actif.

8. Procédé selon l'une des revendications 6 ou 7, les particules de principe actif étant extraites par vaporisation du solvant.

9. Procédé selon l'une des revendications 6 à 8, le clopidogrel et un acide étant mélangés avec la suspension de l'agent d'adsorption.

10. Procédé selon la revendication 6, la dernière étape de la synthèse du clopidogrel ou d'un sel pharmaceutiquement toléré de celui-ci étant effectuée en présence de l'agent d'adsorption.
